# EUROPÄISCHE PATENTSCHRIFT

(11) **EP 2 068 989 B1**
(45) Veröffentlichungstag und Bekanntmachung des Hinweises auf die Patenterteilung: **01.02.2012**
(21) Anmeldenummer: 07820165.4
(22) Anmeldetag: 13.09.2007
(51) Int. Cl.: A61M 16/04

(54) **MANSCHETTE EINER BEATMUNGSVORRICHTUNG**
CUFF OF A RESPIRATORY DEVICE
BALLONNET D'UN DISPOSITIF RESPIRATOIRE

(30) Priorität: 20.09.2006 DE 102006044740
(43) Veröffentlichungstag der Anmeldung: 17.06.2009
(73) Patentinhaber: Tracoe Medical GmbH, 60528 Frankfurt/Main (DE)
(72) Erfinder: SCHNELL, Ralf, 63500 Seligenstadt (DE); WALDECK, Franz, Prof. Dr., 55268 Nieder-Olm (DE)
(74) Vertreter: Lieke, Winfried
(86) Internationale Anmeldenummer: PCT/EP2007/059609
(87) Internationale Veröffentlichungsnummer: WO 2008/034751

(56) Entgegenhaltungen:
- WO-A-2006/002364
- US-A- 4 791 923
- US-A- 5 038 777

## Beschreibung

Die vorliegende Erfindung betrifft die Manschette einer Beatmungsvorrichtung, insbesondere einer Tracheostomiekanüle oder eines Endotrachealtubus, wobei die Manschette einen Beatmungstubus umgreift und abgedichtet an diesem befestigt ist und die Manschette aus einer inneren Folie und einer äußeren Folie besteht, wobei die innere und die äußere Folie voneinander getrennt sind und die äußere Folie aus einem elastisch leicht dehnbaren Material besteht.

Manschetten bei Tracheostomiekanülen oder Endotrachealtuben sind seit langem bekannt. Die Manschetten dienen zum einen dazu, einen Tubus in der Trachea eines Patienten zu fixieren und zu zentrieren, so daß untere Ränder oder Kanten nach Möglichkeit nicht mit der Wand einer Trachea in Eingriff treten. Zum anderen hat die Manschette auch die Funktion, insbesondere bei Patienten, die die Atmung nicht mehr selbst aktiv unterstützen können, ein Entweichen der durch den Tubus zugeführten Atemluft über den Rachenraum nach außen zu verhindern.

Allerdings ist es bekannt, daß insbesondere bei der Langzeitanwendung derartiger Beatmungsvorrichtungen des öfteren Komplikationen auftreten, die unter anderem mit der Manschette zusammenhängen können.

Wie bereits erwähnt, soll die Manschette zum einen verhindern, daß Atemluft, welche durch den Tubus zugeführt wird, nach oben an einem Tubus vorbei durch den Rachenraum wieder entweicht, umgekehrt soll aber die Manschette auch verhindern, daß beispielsweise Sekrete, Flüssigkeit und eventuell auch Speisereste, die über den Rachenraum in die Trachea gelangen und sich oberhalb der Manschette ansammeln können, an der Manschette vorbeitreten können und auf diese Weise in die Bronchien und die Lunge gelangen könnten. Insbesondere sich für längere Zeit oberhalb des Cuffs bzw. der Manschette ansammelndes Sekret kann dabei im Laufe der Zeit auch bakteriologisch kontaminiert sein, so daß das Vorbeitreten derartiger Sekrete an der Manschette bzw. zwischen der Außenwand der Manschette und der Innenwand der Trachea nicht selten zu Lungenentzündungen oder anderen Infektionen führen kann.

Grundsätzlich könnte man zwar zwischen der Außenwand einer Manschette und der Wand der Trachea eine sichere Abdichtung erzielen, wenn man die Manschette mit einem hinreichenden Druck aufbläst, dies führt jedoch dazu, daß die Wand der Trachea einem Druck ausgesetzt wird, der zu einer Schädigung und schließlich zu einer Nekrotisierung des Gewebes führt. Dies versucht man unter anderem durch die Verwendung von Manschetten mit Übermaß zu verhindern, die aus einem im wesentlichen nicht dehnbaren Material bestehen, wobei die mit einem geringen Überdruck von z. B. 20 hPa aufgeblasene Manschette im einschränkungsfreien Zustand einen Durchmesser annimmt, der mit Sicherheit größer ist als der Durchmesser der Trachea, in welcher die betreffende Beatmungsvorrichtung Anwendung findet. Typischerweise liegen die Durchmesser derartiger Manschetten mit Übermaß im aufgeblähten, freien Zustand im Bereich zwischen 20 mm und 35 mm.

Da der Durchmesser einer Trachea jeweils kleiner ist als der Durchmesser einer solchen voll aufgeblähten Manschette, hat die Verwendung derartiger Manschetten den Vorteil, daß man bei einem Aufblähen der Manschette mit einem Druck von z. B. 20 hPa sicher sein kann, daß der von der Manschette ihrerseits auf die Wand der Trachea ausgeübte Druck ebenfalls 20 hPa beträgt, wobei dieser geringe Druck eine Schädigung und Nekrotisierung des Gewebes ausschließt.

Auch wenn sich aufgrund dieses Vorteils die Verwendung von im wesentlichen nicht elastisch dehnbaren Manschetten mit Übermaß weitgehend durchgesetzt hat, haben aber diese Manschetten dennoch den Nachteil, daß sie, da sie sich innerhalb der Trachea nicht auf ihr volles Volumen ausdehnen können, notwendigerweise an ihrem Außenumfang Falten werfen. Diese Falten führen wiederum dazu, daß die Manschette nicht entlang ihres gesamten Umfanges überall dicht an der Wand der Trachea anliegt. Darüber hinaus können sich auch bei eingestülpten Falten Kanäle entlang dieser Falten bilden, durch die Flüssigkeiten bzw. Sekrete, die sich oberhalb der Manschette in einer Trachea ansammeln, an der Manschette vorbei in die Bronchien und die Lunge gelangen können. Dies wird beispielsweise in der DE 198 55 521 ausführlich diskutiert.

Es sind deshalb schon zahlreiche Versuche unternommen worden, die Manschetten in der Weise herzustellen und zu verbessern, daß eine solche Faltenbildung entweder minimiert wird oder die Falten zumindest so klein und eng ausgebildet werden, daß Flüssigkeiten oder Sekrete jedenfalls nicht in nennenswertem Maße zwischen Manschette und Wand der Trachea hindurchtreten können.

Die zur Erzielung dieser Eigenschaften an die Manschette zu stellenden Anforderungen sind allerdings in mancher Hinsicht nicht miteinander und auch nicht mit den konkreten Anwendungsbedingungen verträglich. Stellt man die Manschette aus einem sehr weichen und duktilen Material her, so besteht die Gefahr, daß eine derartige Manschette schon beim Einführen in die Trachea, insbesondere wenn die Manschette an einer Tracheostomiekanüle angeordnet ist und durch ein enges Tracheostoma hindurchgeführt werden muß, einreißt oder in der Weise beschädigt wird, daß sie ihre Funktion nicht mehr erfüllen kann.

Ein entsprechend stabileres und reißfesteres Material ist hingegen in der Regel nicht ausreichend duktil und biegsam, um die entstehenden Falten mit Sicherheit hinreichend klein zu machen, damit Undichtigkeiten vermieden werden.

Man hat auch schon versucht, die Wandstärke des Materials, aus welchem derartige Manschetten bestehen (z. B. Polyurethan) extrem, bis auf Werte unter 10 µm, z. B. 5 µm, zu reduzieren, um dieses Ziel zu erreichen. Auch derart dünnwandige Manschetten sind jedoch relativ empfindlich und schwierig in der Handhabung, insbesondere bei Tracheostomiekanülen. Überdies ist bei so dünnwandigen Manschetten eine ausreichende Dichtigkeit der Manschettenwand gegenüber Wasserdampf nicht mehr gewährleistet, so daß sich in der Manschette Wasser ansammeln kann, wodurch die Funktion der Manschette beeinträchtigt wird.

Weitere Versuche bezüglich einer besseren Abdichtung bestanden im Aufbringen unterschiedlicher Beschichtungen auf die Folie der Manschette, wie beispielsweise in DE 198 55, 521 beschrieben, oder in der Verwendung mehrschichtiger Folien. Dabei sollte die äußere Schicht möglichst' hydrophil oder quellfähig sein um ein Aufsaugen von Flüssigkeit sowie eine gute Anlage an die Trachea zu ermöglichen. Eine solche äußere Schicht kann auch Stoffe enthalten, die ein Bakterienwachstum hemmen oder verhindern. Auch wenn hiermit teilweise gute Ergebnisse erreicht wurden, so kann das nicht lückenlose Anliegen der Manschette an die Trachea dadurch nicht immer vollständig ausgeglichen werden.

Teilweise wurde auch die Verwendung mehrerer Manschetten vorgeschlagen. In DE 196 38 935 und DE 198 45 415 wird vorgeschlagen, oberhalb (cranial) der Manschette eine durch Zufuhr von Fluid vergrößerbare Tamponierblase anzuordnen, die eine zusätzliche abdichtende Funktion hat. Diese Tamponierblase kann sich auch nach kaudal ausdehnen und die Manschette vollständig umschließen. Durch die getrennte Befüllung der Manschette und der Tamponierblase ist in dieser Anordnung eine Steuerung des auf die Tracheawand ausgeübten Drucks nur schwer möglich und eine notwendiges zusätzliches Fluidsystem verkompliziert die Handhabung.

Die US 2004/0236365 offenbart einen Katheter, insbesondere einen Harnröhrenkatheter, bei dem ein Verankerungs- oder Behandlungsballon von einer elastischen Manschette überzogen ist. Diese Manschette dient dem Zusammenpressen der Ballons beim Einführen und Herausnehmen des Katheters, sie übt daher einen starken Druck auf den jeweiligen Ballon aus. Beim Aufblasen der Ballons muß zunächst dieser Widerstand überwunden werden. Es ist daher bei dieser Anordnung sehr schwierig, die tatsächliche Druckbelastung auf die Harnröhre zu bestimmen, wodurch es bei Langzeitanwendungen zur Beschädigung des Gewebes kommen kann.

Trachealkanülen mit den doppelwandig ausgebildeten Manschetten sind aus den Dokumenten US 5,038,777 und US 4,791,923 bekannt.

Der vorliegenden Erfindung liegt daher die Aufgabe zugrunde, eine entsprechende Manschette, sowie eine mit einer solchen Manschette ausgestattete Beatmungsvorrichtung zu schaffen, welche einerseits hinreichend stabil und leicht handhabbar ist, aber keine oder nur sehr kleine, enge Falten entstehen läßt, die die Dichtigkeit des Eingriffs zwischen Manschette und Wand der Trachea auch bei geringen Innendrücken der Manschette von z. B. 20 hPa nicht beeinträchtigen.

Diese Aufgabe wird dadurch gelöst, daß bei einer aus einer inneren Folie und einer davon getrennten äußeren Folie bestehenden Manschette die äußere Folie bei einem Innendruck von höchstens 50 hPa auf einen Durchmesser ausdehnbar ist, der größer ist als der Durchmesser einer Trachea, für die die Beatmungsvorrichtung bestimmt ist, und insbesondere mindestens das 1,5-fache des Außendurchmessers des Tubus beträgt, und die innere Folie aus einem Material mit geringerer elastischer Dehnung besteht, wobei die innere Folie mit Übermaß hergestellt ist und ohne äußere Einschränkung bei einem Innendruck von höchstens 20 hPa einen Durchmesser, der größer ist als der Durchmesser einer Trachea, für welche die Manschette bestimmt ist, annimmt.

Die äußere Folie soll dabei aus einem elastisch leicht dehnbaren Material bestehen, während die innere Folie aus einem Material mit geringerer elastischer Dehnung besteht. Dabei wird unter einem "elastisch leicht dehnbaren Material" ein Material verstanden, welches beispielsweise bei einem Innendruck eines geschlossenen Ballons aus einem solchen Material von 10 hPa bis 15 hPa sich um mindestens 20%, vorzugsweise mindestens 50% oder mehr dehnt.

Im Gegensatz dazu kann die innere Folie zwar möglicherweise auch elastisch dehnbar sein, ist aber auf jeden Fall mit einem Übermaß hergestellt, so daß sie jedenfalls innerhalb der einschränkenden Maße einer Trachea nicht in einen gedehnten Zustand kommen kann und vorzugsweise beispielsweise bei einem Druck von 15 hPa bis 20 hPa sich um weniger als 10%, insbesondere um weniger als 1% dehnt. Das Merkmal, wonach die innere Manschette im Übermaß hergestellt ist und ohne äußere Einschränkung bei einem Innendruck von höchstens 20 hPa einen Durchmesser, der größer ist als der Durchmesser einer Trachea, für welche die Manschette bestimmt ist, annimmt, soll zum Ausdruck bringen, daß der Durchmesser der Folie bzw. inneren Manschette, wenn die Manschette ohne äußere Beschränkung befüllt wird, so daß die Folie faltenfrei ist, größer ist als der Innendurchmesser der Trachea, für die die Manschette bestimmt ist. Bei einer Trachealkanüle für Erwachsene beträgt der Innendurchmesser der Trachea ungefähr das Doppelte des Außendurchmessers des Tubus. Trachealkanülen für Kinder haben in der Regel einen Tubus mit einem relativ größeren Außendurchmesser im Vergleich zum Innendurchmesser der Trachea. Mit Übermaß hergestellte Manschetten sind im Stand der Technik bekannt und werden auch "High-volume-Low-pressure-Manschetten" genannt. Es versteht sich, daß für das Aufblähen einer solchen Manschette schon ein geringer Überdruck von 1-2 hPa ausreichend sein kann, während aber in der Praxis ein Überdruck von ca. 20 hPa verwendet wird um eine derartige Manschette in der Trachea aufzublähen.

Hingegen sollte die äußere Folie sich in einer bevorzugten Ausführungsform der Erfindung bei einem Innendruck von höchstens 50 hPa, vorzugsweise bei höchstens 30 hPa, und besonders bevorzugt zwischen 5 hPa und 30 hPa, elastisch auf einen Durchmesser ausdehnen, der größer ist als der Durchmesser einer Trachea, für welche die Manschette bestimmt ist, und der insbesondere mindestens das Doppelte des Außendurchmessers der Kanüle bzw. des Tubus beträgt.

Derart leicht dehnbare elastische Materialien können in der Praxis nicht als alleiniges Manschettenmaterial verwendet werden, da nämlich in einem solchen Fall nicht klar ist, in welche Richtung die Dehnung des Manschettenmaterials insgesamt stattfindet, d.h. die Manschette dehnt sich nicht unbedingt allein in ihrem Durchmesser so weit aus, daß sie die Wand der Trachea abdichtend berührt, sondern sie kann sich beispielsweise auch in axialer Richtung entsprechend ausdehnen, so daß bei alleiniger Verwendung eines solchen Manschettenmaterials die Anlage an der Wand der Trachea nicht entlang des gesamten Umfanges sichergestellt wäre. Außerdem hat eine solche, elastisch dehnbare Manschette für sich gesehen keine ausreichende Zentrierfunktion und könnte unter Umständen die Öffnung der Trachealkanüle oder des Tubus überdecken.

Die Verwendung einer im wesentlichen nicht dehnbaren inneren Manschette bzw. inneren Folie führt jedoch dazu, daß diese sich in definierter Weise aufgrund der vorgegebenen Form nahezu ausschließlich radial ausdehnt und dabei die leicht elastisch dehnbare äußere Folie ebenfalls im wesentlichen nur in radialer Richtung dehnt. Die sich auf der Außenseite der inneren Folie dabei bildenden Falten werden dann durch das elastisch dehnbare Material der äußeren Folie ohne weiteres überbrückt, die demzufolge eine ideale Form einer Einhüllenden der inneren Folie einnimmt und damit praktisch entlang des gesamten Umfanges glatt und abgedichtet an der Wand der Trachea anliegt.

Da bekannt ist, welcher Druck für eine Dehnung der äußeren Folie in einen solchen Zustand erforderlich ist, muß man den Druck der inneren Manschette lediglich um ein entsprechendes Maß erhöhen, um sicherzustellen, daß die äußere Wand bzw. Folie der Manschette ihrerseits mit einem Druck von ca. 15 hPa bis 20 hPa oder etwas weniger an der Wand der Trachea anliegt. Beispielsweise kann man, wenn 5 hPa bis 10 hPa ausreichen, um den Durchmesser der äußeren, elastisch dehnbaren Folie auf das Zweieinhalbfache zu vergrößern, den Innendruck der inneren Manschette auf etwa 20 hPa bis 30 hPa einstellen, so daß nach Überwindung der nach innen gerichteten elastischen Kraft der äußeren Manschette noch ein Restdruck von etwa 20 hPa auf die Wand der Trachea übertragen wird.

Ein weiterer Vorteil der Manschette, die aus einer inneren Manschette mit Übermaß, die von einer äußeren leicht elastisch dehnbaren Manschette eingehüllt wird, aufgebaut ist, besteht darin, daß selbst bei einer Verletzung der äußeren Folie die innere Manschette wie eine herkömmliche Manschette wirkt und dadurch grundsätzlich noch eine Abdichtung gewährleistet ist.

In einer bevorzugten Ausführungsform ist zwischen der inneren und der äußeren Folie ein Gaspolster vorgesehen. Dieses Gaspolster kann allerdings sehr klein sein. Es verhindert, daß die äußere Folie sich in ihrer Form an die innere Folie anpaßt und womöglich an der inneren Folie "klebt", wodurch wiederum Falten entstehen könnten.

Besonders bevorzugt ist dieses Gaspolster durch die innere Folie und die äußere Folie sowie optional die Außenwand der Kanüle vollständig nach außen begrenzt und abgeschlossen, so daß sich sein Volumen nicht verändert. Beim Befüllen des inneren Ballons wird dadurch sichergestellt, daß sich der Druck, der zum Dehnen der äußeren Folie benötigt wird, nicht durch Schwankungen das Gasvolumens verändert. Dadurch wird eine genaue Angabe des Innendrucks der inneren Manschette zum Erreichen eines bestimmten Drucks (z.B. 20 hPa) auf die Wand der Trachea ermöglicht. Das Gas wird hierbei auch nicht signifikant komprimiert, da es sich in einem zumindest teilweise leicht dehnbaren Raum befindet.

Bevorzugt beträgt das Volumen des Gaspolsters weniger als 20% des Volumens der inneren Manschette in frei befülltem Zustand, d.h. bei Befüllen der Manschette ohne äußere Einschränkungen, bis die innere Folie faltenfrei ist. Unter "innerer Manschette" wird dabei die ausschließlich durch die innere Folie gebildete um den Beatmungstubus angeordnete Manschette verstanden. Unter "Befüllen der Manschette" wird somit das Befüllen der inneren Manschette verstanden. Das Gaspolster soll ein geringes Volumen haben, da der Abstand zwischen innerer und äußerer Folie sowohl im unbefüllten Zustand der Manschette gering sein soll, um ein leichtes Einführen zu ermöglichen, als auch im befüllten Zustand, wodurch die Stabilisierung der äußeren Folie durch die innere Folie erreicht wird.

Weiterhin bevorzugt ist, daß sich zwischen innerer und äußerer Folie eine Substanz befindet, die das Aneinanderhaften der Folien verhindert. Eine solche Substanz kann beispielsweise Talkum oder ein schmierendes Fluid oder Gleitgel (z. B. KY-Jelly) sein. Diese Substanz kann zusätzlich zu einem Gaspolster oder anstelle eines Gaspolsters zwischen der inneren und der äußeren Folie vorhanden sein. Sie stellt sicher, daß die beiden Folien gegeneinander beweglich bleiben und die äußere Folie dadurch die innere Folie insbesondere im aufgeblasenen Zustand mit geringer äußerer Oberfläche umspannt. Ansonsten kann aber auch das Material der beiden Folien so ausgewählt werden, daß sie nur sehr geringe Adhäsionskräfte entwickeln, so daß ein Aneinanderhaften praktisch ausgeschlossen ist.

In einer besonders bevorzugten Ausführungsform weisen die äußere und die innere Folie unterschiedliche Elastizitätsmoduln und/oder Shore-Härten auf, wobei die innere und die äußere Folie grundsätzlich aus demselben Material hergestellt werden können, soweit chemisch gleiche Materialien ohne weiteres mit unterschiedlicher Härte und Elastizität herstellbar sind, wie dies beispielsweise für Polyurethan oder andere thermoplastische Elastomere, insbesondere Copolymere, gilt. Weitere Beispiele für solche Materialien sind thermoplastische Elastomere. Diese Materialien beinhalten im Allgemeinen harte und weiche Segmente.Durch Variation des Verhältnisses von weichen zu harten Segmenten und durch den Gehalt bzw. die Art der Verzweigungen kann der Elastizitäsmodul bzw. die Shore-Härte des Materials über weite Bereiche eingestellt werden.

Weiterhin ist aber auch bevorzugt, für die innere und die äußere Folie verschiedene Materialien zu wählen, die insbesondere zum Erreichen der leichten Dehnbarkeit bei geringer Spannung der äußeren Folie bzw. der Stabilität der inneren Folie vorteilhaft sind. Dabei kommt es lediglich darauf an, daß sowohl die innere als auch die äußere Folie an ihrem oberen bzw. unteren Ende jeweils mit dem Tubus oder auch miteinander fest und abgedichtet verbindbar sind.

Bevorzugt besteht die innere Folie aus einem der Materialien Polyurethan, Polypropylen, Polyethylen, Polyethylenterephthalat, Polyvinylchlorid oder anderen Polymeren und Mischungen aus Polymeren (Blends) und die äußere Folie aus mindestens einem der Polymere Polyurethan, SEBS (Styrol-Ethen-Buten-Styrol), SBS (Styrol-Butadien-Styrol), SIS (Styrol-Isopren-Styrol), IR (Polyisopren) oder anderen thermoplastischen Elastomeren, Latex, Silicon, Naturgummi oder synthetischem Gummi. Unter thermoplastischen Elastomeren sind dabei Materialien zu verstehen, die die Elastizität, Weichheit und Zähigkeit eines wärmehärtenden Kautschuks und die Verarbeitbarkeit eines thermoplastischen Polymers aufweisen. Sie umfassen unter anderem Styrolblockcopolymere, Polyolefingemische, elastomere Legierungen, thermoplastische Polyurethane, thermoplastische Copolyester und thermoplastische Polyamide sowie Mischungen (Blends) daraus. Es sind auch weitere Materialien denkbar, auch solche, die möglicherweise noch entwickelt werden, die die gewünschten Eigenschaften der äußeren oder inneren Folie aufweisen. Selbstverständlich sind die Materialien der inneren und insbesondere der äußeren Folie biokompatibel bzw. körperverträglich, d. h. sie werden durch das Milieu innerhalb des Körpers und insbesondere innerhalb der Trachea nicht beschädigt und schädigen diese auch nicht. Insbesondere künstlicher Latex bzw. Kautschuk, der frei von Allergenen ist, kann verwendet werden.

Mindestens eine der Folien sollte eine wasserdampfundurchlässige Folie sein, wobei es bevorzugt ist, wenn die äußere Folie wasserdampfundurchlässig ist, so daß kein Wasser in den Zwischenraum zwischen äußerer und innerer Folie eindringen kann. Die innere Folie kann wasserdampfundurchlässig sein, so daß sie im Falle einer Verletzung der äußeren Folie zumindest eine eingeschränkte Abdichtfunktion übernehmen kann.

Wird die Manschette, d.h. der Hohlraum der sich zwischen innerer Folie und dem Beatmungstubus befindet, mit einer Flüssigkeit befüllt, so muß wenigstens die innere Folie wasserdampfundurchlässig sein, um ein Durchtreten von Flüssigkeit bzw. Wasserdampf in das Gaspolster zwischen innerer und äußerer Folie zu verhindern.

Zweckmäßigerweise werden beide Folien zumindest entlang eines mit dem Tubus fest und abgedichtet verbundenen Abschnittes auch untereinander fest und dicht verbunden, wobei es aber auch grundsätzlich möglich wäre, die äußere Folie axial oberhalb und unterhalb der Abschnitte mit einem Tubus zu verbinden, entlang welcher die innere Folie mit dem Tubus verbunden ist. Dabei soll die innere Folie zum Lumen der Trachea vollständig durch die äußere Folie abgegrenzt sein.

In einer weiteren Ausführungsform der Erfindung kann die innere und/oder die äußere Folie beschichtet sein. Dabei ist eine Beschichtung eine dünne sich nicht selbsttragende Schicht. Diese Beschichtung kann auf der äußeren Folie zum Beispiel eine nach außen gerichtete hydrophobe oder hydrophile Schicht sein. Eine solche Beschichtung kann auch bakterizide oder bakteriostatische Substanzen enthalten. Denkbar wäre beispielsweise eine Silber oder Silberverbindungen enthaltende Beschichtung. Auch auf den aufeinanderliegenden Folienoberflächen kann eine das Aufeinanderhaften der Folien verhindernde Beschichtung sinnvoll sein.

Bevorzugt bestehen die innere und/oder äußere Folie jeweils aus mehreren fest miteinander verbundenen Folienschichten. Insbesondere die äußere Folie, die einerseits elastisch leicht dehnbar andererseits möglichst dicht gegenüber Wasserdampf und Wasser sein soll, kann diese Eigenschaften durch zwei unterschiedliche Folienmaterialien erhalten, die als dünne Folienschichten miteinander verbunden sind. Soweit die Folienschichten aus unterschiedlichen und schwer miteinander verbindbaren Materialien bestehen, kann für die Verbindung der Folienschichten ein sogenannter Verträglichkeitsvermittler verwendet werden. Solche Verträglichkeitsvermittler sind z. B. Copolymere, insbesondere Diblockcopolymere. Falls die zu verwendenden Materialien dies zulassen, können die Folienschichten der einzelnen Folien der Manschette auch miteinander koextrudiert oder aufeinander laminiert sein.

Zweckmäßig beträgt die Wandstärke der inneren Folie zwischen 20 µm und 100 µm, bevorzugt zwischen 50 µm und 100 µm. Dieser Wandstärkebereich ermöglicht eine ausreichende Stabilisierung der äußeren Folie bei gleichzeitiger ausreichender Flexibilität und Anpaßbarkeit der inneren Folie, wobei die Größe der sich bei der inneren Folie ausbildenden Falten keine Rolle spielt, da diese von der äußeren Folie überbrückt werden.

Die Wandstärke der äußeren Folie beträgt im drucklosen Zustand sinnvollerweise zwischen 20 µm und 600 µm, bevorzugt zwischen 50 µm und 400 µm und besonders bevorzugt zwischen 100 µm und 300 µm. Die äußere Folie wird insbesondere beim Einführen der Kanüle oder des Tubus durch Scherkräfte belastet und muß daher eine ausreichende Wandstärke haben, um dieser mechanischen Belastung standzuhalten. Andererseits muß die Folie hinreichend elastisch dehnbar sein, um beim Befüllen der Manschette keinen großen Widerstand zu verursachen und eine optimale Anpassung an die Wand der Trachea zu gewährleisten.

Eine bevorzugte Ausführungsform einer Manschette bzw. einer Beatmungsvorrichtung mit einem Tubus ist dadurch gekennzeichnet, daß die äußere und die innere Folie im wesentlichen die gleiche axiale Länge haben und sich im wesentlichen über den gleichen axialen Bereich des Tubus erstrecken, wobei äußere und innere Folie jeweils an denselben axialen Positionen an dem Tubus bzw. aufeinander abgedichtet befestigt sind. Dies trägt dazu bei, daß zwischen äußerer und innerer Folie kein oder nur ein sehr geringer Zwischenraum verbleibt, was verhindert, daß sich Teile der sehr elastischen äußeren Folie gegenüber der inneren Folie nennenswert verschieben.

Weitere Vorteile, Merkmale und Anwendungsmöglichkeiten der vorliegenden Erfindung werden deutlich anhand der folgenden Beschreibung einer bevorzugten Ausführungsform und der dazugehörigen Figuren.

Es zeigen:
- Figur 1:: eine schematische Darstellung einer Tracheostomiekanüle mit einer Ausführungsform einer erfindungsgemäßen Manschette,
- Figur 2:: einen Längsschnitt einer Luftröhre (Trachea) mit einer Tracheostomiekanüle mit einer Ausführungsform einer erfindungsgemäßen Manschette im befüllten Zustand,
- Figur 3:: einen Querschnitt durch einen Beatmungstubus mit einer erfindungsgemäßen Manschette, die an einer nur andeutungsweise dargestellten Tracheawand anliegt und
- Figur 4:: einen vergrößerten Schnitt durch die an die Tracheawand anliegende Manschette, der dem Kreis A in Figur 3 entspricht.

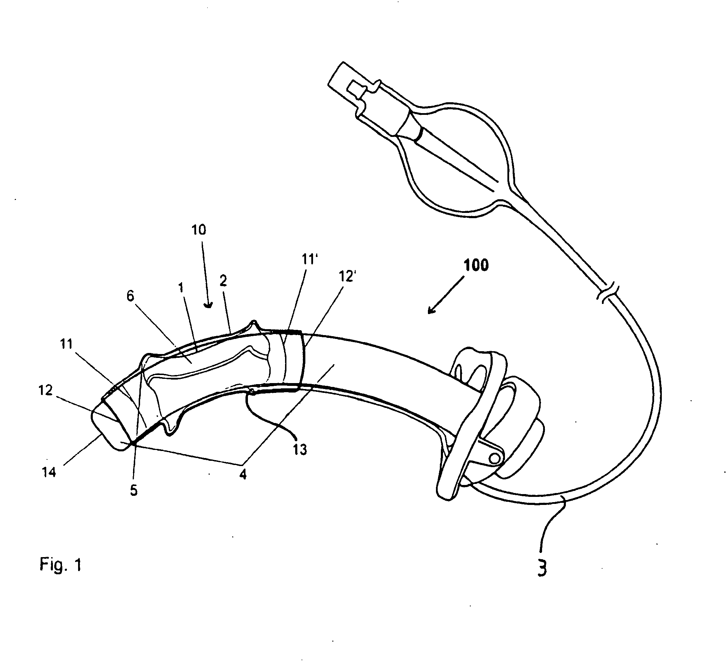

In Figur 1 erkennt man eine Trachealkanüle 100 mit einer Manschette 10 in einem teilweise aufgeblasenen Zustand. Wenn durch Entleeren der inneren Manschette sich innere und äußere Folie eng an den Tubus anlegen, kann die Trachealkanüle in die Trachea eingeführt werden. Die Manschette 10 dieser Kanüle besteht aus einer inneren Folie 1, die an den Verbindungsstellen 11 und 11' mit dem Beatmungstubus 4 der Trachealkanüle 100 verbunden ist, und einer äußeren Folie 2, die an den Verbindungsstellen 12 und 12' mit dem Beatmungstubus 4 verbunden ist. Die Verbindungsstellen 11, 11', 12 und 12' sind jeweils so gestaltet, daß sie luft- bzw. gasundurchlässig sind.

Die innere Folie 1 entspricht einer sogenannten "High-volume-Low-pressure-Manschette", die mit Übermaß hergestellt ist und im vollständig entfalteten befüllten Zustand einen größeren Durchmesser senkrecht zu dem Beatmungstubus 4 hat als der Durchmesser der Trachea, für die die Manschette bestimmt ist. Die äußere Folie 2 besteht aus einem elastisch sehr leicht dehnbaren Material, sie ist bei sehr geringem Innendruck von weniger als 30 hPa auf einen Durchmesser ausdehnbar, der größer ist als der Durchmesser einer Trachea, für die die Manschette bestimmt ist. Aufgrund der sehr leichten Dehnbarkeit wäre die äußere Folie 2 alleine nicht als sogenannte "Low-volume-High-pressure-Manschette" einsetzbar, da sie eine zu geringe Formstabilität hat und dadurch möglicherweise beim Befüllen die Öffnung 14 des Beatmungstubus 4 verschließen könnte.

Die innere Manschette ist aus für "High-volume-Low-pressure-Manschetten" üblichen Materialien hergestellt. Die äußere Manschette besteht aus SIS, SBS, SEBS, anderen thermoplastischen Elastomeren oder einer Mischung daraus, die mit den gewünschten Eigenschaften, z. B. von der Fa. Kraton, erhältlich sind. Bei einer Wandstärke der äußeren Manschette von z.B. 100 µm bis 300 µm lassen die vorgenannten Materialien sich ohne weiteres so einstellen; z. B. für SBS in dem Verhältnis von Styrol zu Butadien, daß sich eine daraus gefertigte Manschette bei einem Druck zwischen 5 hPa und 30 hPa in ihrem Durchmesser gegenüber dem drucklosen Zustand mindestens verdoppelt beziehungsweise auf einen Durchmesser ausdehnt, der mindestens dem doppelten Durchmesser des Tubus entspricht.

Zwischen innerer und äußerer Manschette befindet sich eine geringe Menge Gas 5, z. B. Luft oder auch CO₂ oder N₂, die dazu dient, die beiden Folien getrennt zu halten, deren Volumen allerdings so gering ist, daß die äußere Folie 2, in entleertem Zustand der inneren Manschette, den Beatmungstubus 4 und die innere Folie 1 eng umgreift. Wahlweise befindet sich anstelle des Gases ein Gleitgel oder einer Mischung aus Gas und Gleitgel.

Leicht erkennbar ist der Schlauch 3 in Figur 1, der die Zufuhr eines Fluids, z.B. Luft, in den durch die innere Folie 1 und den Beatmungstubus 4 abgeschlossenen Hohlraum 6 ermöglicht. Das Fluid tritt dabei durch die Öffnung 13 in den Hohlraum 6.

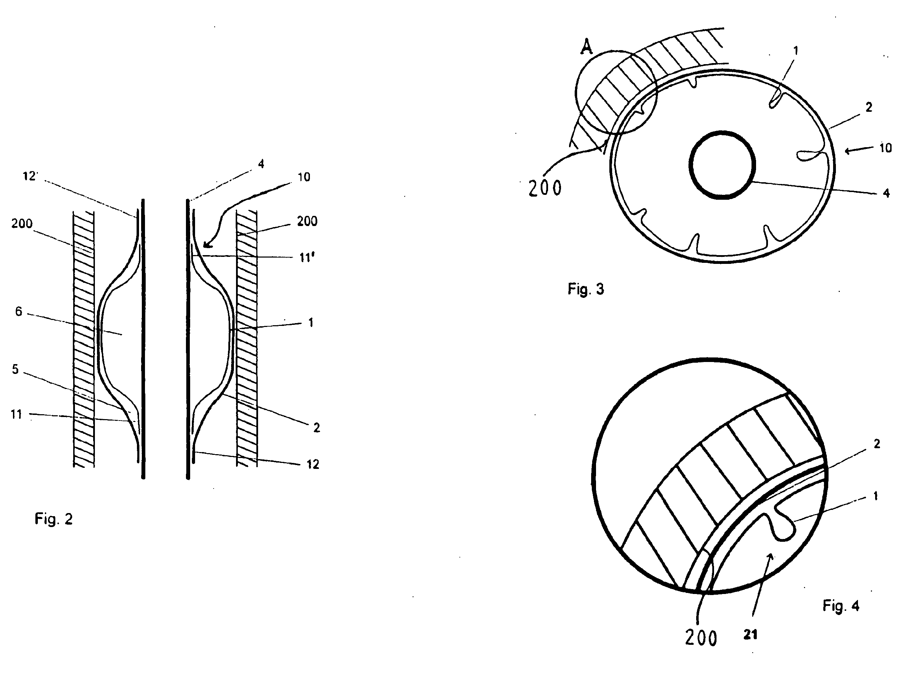

In Figur 2 ist schematisch ein Schnitt durch eine Trachea gezeigt, in der sich ein Beatmungstubus 4 mit einer Manschette 10 befindet. Die Manschette 10, beziehungsweise der Hohlraum 6 zwischen innerer Folie 1 und Beatmungstubus 4, ist mit einem Fluid gefüllt. Dadurch ist die innere Manschette soweit ausgedehnt, daß sie die größtmögliche Auflagefläche mit der Tracheawand 200 ausbilden würde. Zwischen der inneren Folie 1 und der Tracheawand 200 befindet sich die äußere Folie 2, die durch das Befüllen der Manschette gedehnt wurde. Die äußere Folie 2 liegt faltenfrei an der Tracheawand 200 und überbrückt dadurch die von der inneren Folie 1 gebildeten Falten.

Zwischen der inneren Folie 1 und der äußeren Folie 2 sowie dem Beatmungstubus 4 befindet sich ein Gaspolster 5, das durch die Verbindungsstellen 11, 11', 12 und 12' der beiden Folien mit dem Beatmungstubus abgedichtet ist. Das Volumen diese Gaspolsters bleibt daher beim Befüllen der Manschette praktisch unverändert. Dieses Polster ermöglicht eine fast faltenfreie Oberfläche der äußeren Folie im nichtbefüllten Zustand der Manschette. Beim Befüllen wird durch dieses Polster ein Aneinanderkleben der inneren Folie und der äußeren Folie verhindert, was letztlich zu dem faltenfreien Anliegen der äußeren Folie 2 der Manschette 10 an die Tracheawand 200 führt.

In Figur 3 ist schematisch ein Querschnitt durch einen Beatmungstubus 4 im Bereich der Manschette 10 dargestellt. Die Manschette ist dabei befüllt, so daß sie der Fixierung der Tracheostomiekanüle oder des Endotrachealtubus dient. Die innere Manschette ist befüllt und der Fülldruck ist so eingestellt, daß unter Berücksichtigung der nach innen gerichteten Rückstellkraft der äußeren Manschette (Folie) die gesamte Manschette 10 an der Tracheawand 200 mit einem Druck von etwa 20 hPa anliegt. Aufgrund der räumlichen Einschränkung der Manschette 10 durch die Tracheawand 200 bilden sich in der inneren Folie 1 Falten, die von der glatt anliegenden äußeren Folie 2 jedoch überbrückt werden, so daß die gesamte Manschette 10 glatt an der Tracheawand anliegt.

Zur Verdeutlichung des Kontakts zwischen Tracheawand 200 und Manschette 10 ist in Figur 4 der Ausschnitt A aus Figur 3 vergrößert dargestellt. Bei der Manschette in befülltem Zustand innerhalb der Trachea bilden sich bei der inneren Folie 1 Falten 21 aus. Die innere Manschette spannt dabei die äußere Manschette, die durch die Folie 2 gebildet wird. Die äußere Manschette liegt dadurch faltenfrei an der Tracheawand 200 an und kann sich durch ihre Elastizität auch möglichen Unebenheiten der Tracheawand anpassen. Durch die leichte elastische Dehnbarkeit der äußeren Manschette, die selbst nicht zusätzlich befüllt wird, läßt sich mit Hilfe der Befüllung der inneren Manschette der Druck der durch die gesamte Manschette 10 auf die Tracheawand 200 ausgeübt wird einstellen.

Für Zwecke der ursprünglichen Offenbarung wird darauf hingewiesen, daß sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

## Patentansprüche

1. Manschette (10) einer Beatmungsvorrichtung, insbesondere einer Tracheostomiekanüle (100) oder eines Endotrachealtubus, wobei die Manschette (10) ausgebildet ist, einen Beatmungstubus (4) zu umgreifen und abgedichtet an diesem befestigt zu sein und die Manschette aus einer inneren Folie (1) und einer äußeren Folie (2) besteht, wobei die innere und die äußere Folie voneinander getrennt sind und die äußere Folie aus einem elastisch leicht dehnbaren Material besteht, **dadurch gekennzeichnet, daß** die äußere Folie bei einem Innendruck von höchstens 50 hPa auf einen Durchmesser ausdehnbar ist, der größer ist als der Durchmesser einer Trachea, für die die Beatmungsvorrichtung bestimmt ist, und insbesondere mindestens das 1,5-fache des Außendurchmessers des Tubus beträgt, und die innere Folie aus einem Material mit geringerer elastischer Dehnung besteht, wobei die innere Folie mit Übermaß hergestellt ist und ohne äußere Einschränkung bei einem Innendruck von höchstens 20 hPa einen Durchmesser, der größer ist als der Durchmesser einer Trachea, für welche die Manschette bestimmt ist, annimmt.

2. Manschette nach Anspruch 1, **dadurch gekennzeichnet, daß** zwischen innerer (1) und äußerer Folie (2) ein Gaspolster (5) vorgesehen ist.

3. Manschette nach einem der Ansprüche 1 oder 2, **dadurch gekennzeichnet, daß** das Gaspolster (5) ein zwischen dem äußeren und dem inneren Ballon nach außen abgeschlossener Bereich ist.

4. Manschette nach einem der Ansprüche 1 bis 3, **dadurch gekennzeichnet, daß** das Volumen des Gaspolster (5) weniger als 20% des Volumens der inneren Manschette in frei befülltem Zustand darstellt, insbesondere weniger als 1 ml.

5. Manschette nach einem der vorangehenden Ansprüche, **dadurch gekennzeichnet, daß** zwischen innerer (1) und äußerer (2) Folie eine Substanz, beispielsweise ein Gleitgel, eingebracht ist, die eine Haftung der beiden Folien aneinander verhindert.

6. Manschette nach einem der vorangehenden Ansprüche, **dadurch gekennzeichnet, daß** die innere (1) und die äußere (2) Folie unterschiedliche Elastizitätsmoduln und/oder Shore-Härten aufweisen.

7. Manschette nach einem der vorangehenden Ansprüche, **dadurch gekennzeichnet, daß** die innere (1) und die äußere Folie (2) aus unterschiedlichen Materialien bestehen.

8. Manschette nach einem der vorangehenden Ansprüche, **dadurch gekennzeichnet, daß** die innere Folie (1) aus einem der Materialien Polyurethan, Polypropylen, Polyethylen, Polyethylenterephthalat, Polyvinylchlorid oder anderen Polymeren und Mischungen aus Polymeren und die äußere Folie (2) aus mindestens einem der Polymere Polyurethan, SEBS (Styrol-Ethen-Buten-Styrol), SBS (Styrol-Butadien-Styrol), SIS (Styrol-Isopren-Styrol), IR (Polyisopren) oder anderen thermoplastischen Elastomeren, Latex, Silicon, Naturgummi oder synthetischem Gummi besteht.

9. Manschette nach einem der vorangehenden Ansprüche, **dadurch gekennzeichnet, daß** die äußere Folie (2) und/oder die innere Folie (1) eine wasserdampfundurchlässige Folie ist.

10. Manschette nach einem der vorangehenden Ansprüche, **dadurch gekennzeichnet, daß** beide Folien mindestens entlang eines mit dem Tubus (4) fest und abgedichtet verbundenen Abschnittes (11, 12, 11', 12') ihrerseits fest und dicht miteinander verbunden sind.

11. Manschette nach einem der vorangehenden Ansprüche, **dadurch gekennzeichnet, daß** die äußere Folie (2) und/oder die innere Folie (1) mit einer Beschichtung versehen ist.

12. Manschette nach einem der vorangehenden Ansprüche, **dadurch gekennzeichnet, daß** die äußere Folie (2) und/oder die innere Folie (1) aus mehreren fest miteinander verbundenen Folienschichten bestehen.

13. Manschette nach einem der vorangehenden Ansprüche, **dadurch gekennzeichnet, daß** die Wandstärke der inneren Folie (1) eine Wandstärke zwischen 20 µm und 100 µm, vorzugsweise zwischen 50 µm und 100 µm beträgt.

14. Manschette nach einem der vorangehenden Ansprüche, **dadurch gekennzeichnet, daß** die Wandstärke der äußeren Folie (2) zwischen 20 µm und 600 µm, vorzugsweise zwischen 50 µm und 400 µm und insbesondere zwischen 100 µm und 300 µm beträgt.

15. Manschette nach einem der vorangehenden Ansprüche, **dadurch gekennzeichnet, daß** die äußere (2) und die innere Folie (1) sich im wesentlichen über den gleichen axialen Bereich erstrecken, wobei äußere und innere Folie gemeinsam an denselben axialen Positionen an einem Tubus bzw. aufeinander abgedichtet befestigt sind.

16. Endotracheal- oder Tracheotomietubus, **dadurch gekennzeichnet, daß** er eine Manschette nach einem der Ansprüche 1 bis 15 aufweist.

## Claims

1. A collar (10) of a respiratory device, in particular a tracheotomy cannula (100) or an endotracheal tube, wherein the collar (10) is designed to embrace a respiratory tube and to be fixed thereto in sealing relationship and the collar comprises an inner film (1) and an outer film (2), wherein the inner and the outer films are separate from each other and the outer film comprises an elastically easily stretchable material, **characterised in that** the outer film is expandable at an internal pressure of at most 50 hPa to a diameter which is larger than the diameter of a trachea for which the respiratory device is intended, and in particular is at least 1.5 times the outside diameter of the tube, and the inner film comprises a material of lower elastic stretching, wherein the inner film is produced with oversize and without an external restriction at an internal pressure of at most 20 hPa assumes a diameter which is larger than the diameter of a trachea for which the collar is intended.

2. A collar as set forth in claim 1 **characterised in that** a gas cushion (5) is provided between the inner film (1) and the outer film (2).

3. A collar as set forth in one of claims 1 and 2 **characterised in that** the gas cushion (5) is a region which is closed off outwardly between the outer and the inner balloons.

4. A collar as set forth in one of claims 1 through 3 **characterised in that** the volume of the gas cushion (5) represents less than 20% of the volume of the inner collar in the freely filled condition, in particular less than 1 ml.

5. A collar as set forth in one of the preceding claims **characterised in that** a substance, for example a lubricant gel, which prevents the two films from adhering to each other, is introduced between the inner film (1) and the outer film (2).

6. A collar as set forth in one of the preceding claims **characterised in that** the inner film (1) and the outer film (2) have different moduli of elasticity and/or Shore hardnesses.

7. A collar as set forth in one of the preceding claims **characterised in that** the inner film (1) and the outer film (2) comprise different materials.

8. A collar as set forth in one of the preceding claims **characterised in that** the inner film (1) comprises one of the materials polyurethane, polypropylene, polyethylene, polyethylene terephthalate, polyvinyl chloride or other polymers and mixtures of polymers and the outer film (2) comprises at least one of the polymers polyurethane, SEBS (styrene-ethene-butene-styrene), SBS (styrene-butadiene-styrene), SIS (styrene-isoprene-styrene), IR (polyisoprene) or other thermoplastic elastomers, latex, silicone, natural rubber or synthetic rubber.

9. A collar as set forth in one of the preceding claims **characterised in that** the outer film (2) and/or the inner film (1) is a water vapor-impermeable film.

10. A collar as set forth in one of the preceding claims **characterised in that** at least along a portion (11, 12, 11', 12') which is fixedly and sealingly connected to the tube (4) both films are in turn fixedly and sealingly connected together.

11. A collar as set forth in one of the preceding claims **characterised in that** the outer film (2) and/or the inner film (1) is provided with a coating.

12. A collar as set forth in one of the preceding claims **characterised in that** the outer film (2) and/or the inner film (1) comprise a plurality of film layers which are fixedly connected together.

13. A collar as set forth in one of the preceding claims **characterised in that** the wall thickness of the inner film (1) is a wall thickness of between 20 µm and 100 µm, preferably between 50 µm and 100 µm.

14. A collar as set forth in one of the preceding claims **characterised in that** the wall thickness of the outer film (2) is between 20 µm and 600 µm, preferably between 50 µm and 400 µm and in particular between 100 µm and 300 µm.

15. A collar as set forth in one of the preceding claims **characterised in that** the outer film (2) and the inner film (1) extend substantially over the same axial region, wherein the outer and the inner films are jointly fixed in sealed relationship at the same axial positions to a tube and to each other respectively.

16. An endotracheal or tracheotomy tube **characterised in that** it has a collar as set forth in one of claims 1 through 15.

## Revendications

1. Ballonnet (10) d'un dispositif respiratoire, en particulier d'une canule de trachéotomie (100) ou d'un tube endotrachéal, le ballonnet (10) étant conçu pour envelopper un tube respiratoire (4) et être fixé à celui-ci de manière étanche, le ballonnet étant constitué d'une feuille intérieure (1) et d'une feuille extérieure (2), la feuille intérieure et la feuille extérieure étant séparées l'une de l'autre et la feuille extérieure étant constituée d'un matériau facilement extensible de manière élastique, **caractérisé en ce que** la feuille extérieure peut être étirée dans le cas d'une pression intérieure d'au plus 50 hPa, jusqu'à un diamètre supérieur à celui d'une trachée pour laquelle le dispositif respiratoire est destiné, représentant en particulier au moins 1,5 fois le diamètre extérieur du tube, la feuille intérieure étant constituée d'un matériau possédant un allongement élastique plus faible, la feuille intérieure étant fabriquée de manière surdimensionnée et atteignant, sans restriction extérieure et à une pression intérieure d'au plus 20 hPa, un diamètre supérieur au diamètre d'une trachée pour laquelle le ballonnet est destiné.

2. Ballonnet, selon la revendication 1, **caractérisé en ce qu'** un matelas de gaz (5) est prévu entre la feuille intérieure (1) et la feuille extérieure (2).

3. Ballonnet, selon la revendication 1 ou 2, **caractérisé en ce que** le matelas de gaz (5) constitue un domaine fermé vers l'extérieur, entre le ballon extérieur et le ballon intérieur.

4. Ballonnet, selon l'une des revendications 1 à 3, **caractérisé en ce que** le volume du matelas de gaz (5) représente moins de 20% du volume du ballonnet intérieur dans un état non entièrement rempli, en particulier inférieur à 1 ml.

5. Ballonnet, selon l'une des revendications précédentes, **caractérisé en ce qu'** une substance, par exemple un lubrifiant, est introduise entre la feuille intérieure (1)et la feuille extérieure (2), cette substance empêchant une adhérence mutuelle des deux feuilles.

6. Ballonnet selon l'une des revendications précédentes, **caractérisé en ce que** la feuille intérieure (1) et la feuille extérieure (2) comportent différents modules d'élasticité et/ou de dureté Shore.

7. Ballonnet selon l'une des revendications précédentes, **caractérisé en ce que** la feuille intérieure (1) et la feuille extérieure (2) sont composées de matériaux différents.

8. Ballonnet selon l'une des revendications précédentes, **caractérisé en ce que** la feuille intérieure (1) est composée d'un des matériaux polyuréthane, polypropylène, polyéthylène, polyéthylène téréphtalate, polychlorure de vinyle ou autres polymères ou mélange de polymères, et **en ce que** la feuille extérieure (2) se compose d'au moins un des polymères polyuréthane, SEBS (styrène-éthène-butène-styrène), SBS (styrène-butadiène-styrène), SIS (styrène-isoprène-styrène), IR (polyisoprène) ou autres élastomères thermoplastiques, latex, silicone, caoutchouc naturel ou autre caoutchouc synthétique.

9. Ballonnet selon l'une des revendications précédentes, **caractérisé en ce que** la feuille extérieure (2) et/ou la feuille intérieure (1) est une feuille perméable à l'eau et à la vapeur d'eau.

10. Ballonnet selon l'une des revendications précédentes, **caractérisé en ce que** les deux feuilles sont de leur coté liées entre elles de manière ferme et étanche au minimum le long d'un segment (11, 12, 11',12') lié au tube (4) de manière ferme et étanche.

11. Ballonnet selon l'une des revendications précédentes, **caractérisé en ce que** la feuille extérieure (2) et/ou la feuille intérieure (1) est (sont) munie(s) d'un revêtement.

12. Ballonnet selon l'une des revendications précédentes, **caractérisé en ce que** la feuille extérieure (2) et/ou la feuille intérieure (1) sont(est) composée(s) de plusieurs couches de feuille fermement liées les unes aux autres.

13. Ballonnet selon l'une des revendications précédentes, **caractérisé en ce que** l'épaisseur de paroi de la feuille intérieure (1) représente une épaisseur de paroi entre 20 et 100 µm, de préférence entre 50 et 100 µm.

14. Ballonnet selon l'une des revendications précédentes, **caractérisé en ce que** l'épaisseur de paroi de la feuille extérieure (2) représente une épaisseur de paroi entre 20 et 600 µm, de préférence entre 50 et 400 µm, en particulier entre 100 et 300 µm.

15. Ballonnet selon l'une des revendications précédentes, **caractérisé en ce que** la couche extérieure (2) et la couche intérieure (1) s'étendent essentiellement sur le même domaine axial, la couche extérieure et celle intérieure étant fixées ensemble sur les mêmes positions axiales contre un tube ou de manière étanche l'une sur l'autre.

16. Tube endotrachéal ou de trachéotomie **caractérisé en ce qu'**il présente un ballonnet selon l'une des revendications 1 à 15.
